# EUROPEAN PATENT APPLICATION

(11) **EP 1 627 913 A1**
(43) Date of publication of application: **22.02.2006**
(21) Application number: 04735372.7
(22) Date of filing: 28.05.2004
(51) Int. Cl.: C12N 5/06, C12Q 1/02

(54) **MESENCHYMAL STEM CELL**

(30) Priority: 28.05.2003 JP 2003150690; 26.04.2004 JP 2004129857
(71) Applicant: RIKEN, Wako-shi, Saitama 351-0198 (JP)
(72) Inventor: NISHIKAWA, Shinichi, Kobe-shi, Hyogo 650-0003 (JP); ERA, Takumi, Kobe-shi, Hyogo 658-0084 (JP)
(74) Representative: Schlich, George William
(86) International application number: PCT/JP2004/007735
(87) International publication number: WO 2004/106502

(57) **Abstract**

It is intended to obtain a mesenchymal stem cell in the early stage of differentiation from a pluripotent stem cell differentiated in vitro. A mesenchymal stem cell is obtainable by: a) the step of culturing pluripotent stem cells, b) the step of confirming the appearance of a cell with stroma cell-like morphology, and c) the step of selecting and separating a PDGFR α-positive and FLK1-negative cell.

## Description

### TECHNICAL FIELD

The present invention relates to a mesenchymal stem cell differentiated from pluripotent stem cells in vitro, and to a method of preparing such a stem cell.

### BACKGROUND ART

In modern society the number of people suffering from lifestyle-related disorders such as diabetes and arteriosclerotic conditions including ischemic heart disease and cerebrovascular disease is increasing rapidly. One risk factor for lifestyle-related diseases is obesity, and accumulation of visceral fat in particular. It has recently been discovered that fat tissue is an extremely important tissue for systemic sugar and lipid metabolism, excreting a variety of bioactive substances such as hormones and cytokines in response to changes in the body's internal energy balance and morphological changes in the fat cells themselves. Consequently, analysis has been actively directed at differentiation of fat cells, but most analysis has focused on the final stage of differentiation using cultured cells lines such as 3T3L1 cells, which are fat precursor cells, and there has been no progress in analyzing the differentiation at early stage (Francine M. Gregoire, *Experimental Biology and Medicine* 226, 997-1002, 2001; and Christy, R.J. et al, *Genes and Development* 3, 1323-1335, 1989).

Osteoblasts, which is a general term for precursor cells before differentiation into bone cells, are mononuclear cells with bone-forming ability which have differentiated from osteogenic, undifferentiated mesenchymal stem cells (Shinichi Harada et al, *Nature* 423, 349-355, 2003). The regulatory function of osteoblast differentiation from undifferentiated mesenchymal stem cells is unknown, but bone morphogenetic proteins (BMP) may be involved.

Embryonic stem cells (ES cells) are pluripotent cells in early embryos, and when injected into other blastocytes are capable of differentiating into a variety of cells including reproductive cells. Mouse embryonic stem cells, which have been the most studied, are pluripotent, autoreproducing cells which have been established from the inner cell mass within the blastula at 3.5 days of development. With the simple addition of serum and a growth factor called leukemia inhibitory factor (LIF) to normal growth media, these cells have the ability to maintain proliferation while remaining in an undifferentiated state. When mouse stem cells are injected into blastulae at 3.5 days of development and the blastulae are returned to the parent, they are capable of differentiating into all other tissue cells in vivo, and they are used in preparing chimera mice and knock-out mice. In recent years, it has become possible to manipulate embryonic stem cells in vitro, differentiating them into a variety of mature tissue cells. Because of their pluripotency and ease of manipulation, such embryonic stem cells offer promise for future medicine as materials for transplantation therapies using cells.

The researches of these inventors and other groups have shown that mature cells appear when embryonic stem cells are forced to differentiate in vitro (for example, Shinichi Nishikawa et al, *Development* 125, 1747-1757, 1998; Toru Nakano et al, *Science* 265, 1098-1101, 1994; Takumi Era et al, *Blood* 95, 870-878, 2000). At present it is thought that embryonic stem cells become stem cells at various stage of differentiation before becoming mature cells, but much is still unknown about the differentiation process. A method has been reported in which embryonic stem cells are made to differentiate efficiently into fat cells by first forming embryoid bodies (C. Dani et al, *Journal of Cell Science* 110, 1279-1285, 1997). Differentiation into cartilage cells is described in Naoki Nakayama et al, *Journal of Cell Science* 116, 2015-2028, 2003.

### DISCLOSURE OF THE INVENTION

It is an object of the present invention to obtain a mesenchymal stem cell at an early stage of differentiation from pluripotent stem cells differentiated in vitro.

As a result of exhaustive research, the inventors discovered that when embryonic stem cells are cultured in vitro, mesenchymal stem cells appear at an early stage of differentiation, and that this cell population expresses two special cell surface markers, platelet-derived growth factor receptor α (PDGFRα) and fetal liver kinase-1 (FLK1) in a particular way, and they perfected the present invention based on these findings.

The present invention provides a mesenchymal stem cell that has been differentiated from pluripotent stem cells in vitro. This cell, which has a stroma cell-like morphology, is PDGFRα positive and FLK1 negative, and does not express Mesp2. This cell may be capable of differentiating into a fat cell, osteoblast and/or cartilage cell.

The present invention provides a method of preparing mesenchymal stem cells comprising the following steps.
a) culturing pluripotent stem cells,
b) confirming the appearance of cells with stroma cell-like morphology, and
c) selecting and segregating cells that are PDGFRα positive and FLK1 negative.

In step a) of this method, a process may be included to differentiate the pluripotent stem cells into mesenchymal stem cells.

The invention also provides methods of differentiating mesenchymal stem cells obtainable by the aforementioned method into fat cells, osteoblasts or cartilage cells, as well as fat cells, osteoblasts and cartilage cells obtainable by these methods.

### BRIEF DESCRIPTION OF THE DRAWINGS

Figure 1 shows the progress of PDGFRα expression after differentiation induction with RA.
Figures 2 A-C show the progress of PDGFRα expression after induction of differentiation into fat cells.
Figures 3 A-C show differentiation of PDGFRα-positive and -negative cells into fat cells.
Figure 4 shows that RA(-) 4-day PDGFRα-positive, FLK1-negative cells do not differentiate into fat cells.
Figure 5 shows the marker gene expression patterns of RA(-) 4-day and RA(+) 9-day PDGFRα-positive, FLK1-negative cells.
Figure 6 is a graph comparing fat cell differentiation efficiency depending on time of RA addition.
Figure 7 shows the autoreproducing ability of PDGFRα-positive, FLK1-negative cells selected and separated on the ninth day.
Figure 8 shows that PDGFRα-positive, FLK1-negative cells which have been subcultured 30 times have the ability to differentiate into fat cells.
Figure 9 shows differentiation of PDGFRα-positive and - negative cells into osteoblasts and cartilage cells.
Figure 10 illustrates the cloning process for PDGFRα-positive, FLK1-negative cells.
Figure 11 shows that all cells in the cloned cell line are PDGFRα positive.
Figure 12 shows that PDGFRα-positive, FLK1-negative cells differentiate into both fat cells and osteoblasts.

### BEST MODE FOR CARRYING OUT THE INVENTION

In these specifications, "mesenchymal cells" are osteoblasts, cartilage cells, myoblasts, fat cells, stroma cells, tendon cells and other cells that form mesenchymal tissue, and mesenchymal stem cells which may differentiate into these. Mesenchymal cells occurring during embryogenesis, mesenchymal cells in individual animals, and mesenchymal cells generated by differentiation from pluripotent stem cells in vitro or in vivo are all included in the term "mesenchymal cells".

In these specifications, a "mesenchymal stem cell" is a mesenchymal cell having the ability to autoreproduce and to differentiate into one or more mesenchymal cells. Like a mesodermal cell, a mesenchymal stem cell is pluripotent, capable of differentiating into osteoblasts, cartilage cells, myoblasts, fat cells, stroma cells, tendon cells and the like. While autoreproducing and pluripotent mesodermal cells lose these abilities in the process of development, mesenchymal stem cells are known to persist for a long time in the adult body after it has passed through the development stage. Consequently, mesenchymal stem cells are expected to be useful in cell transplantation therapy.

In these specifications, "in vitro" means that a reaction or culture is performed outside the living body, including the embryo. Various media, reagents and containers suited to the growth and development of cells can be used for culturing and/or differentiating cells in vitro. In these specifications, "in vivo" means that a reaction or culture is performed inside the living body, including the embryo, and that all phenomena occur in the living body.

In these specifications "pluripotent stem cells" are autoreproducible stem cells which are capable of differentiating into at least two selected from the ectodermic, mesodermal and endodermic cells, and include embryonic stem cells, embryonic germ (EG) cells, embryonic carcinoma (EC) cells, multipotent adult progenitor (MAP) cells, adult pluripotent stem (APS) cells, bone marrow stem cells and the like. Pluripotent stem cells derived from a variety of animals including humans, monkeys, mice, rats, hamsters, rabbits, guinea pigs, cows, pigs, dogs, horses, cats, goats, sheep and other mammals, birds, reptiles and the like can be used, but normally mammalian cells are used.

In these specifications, an "embryonic stem cell" is a pluripotent cell present in the early embryo that can differentiate into a variety of cells including reproductive cells when injected into another blastocyst. In the present invention, it is possible to use either embryonic stem cells that have been newly established from the internal cell mass inside a blastula, or an already established cell line.

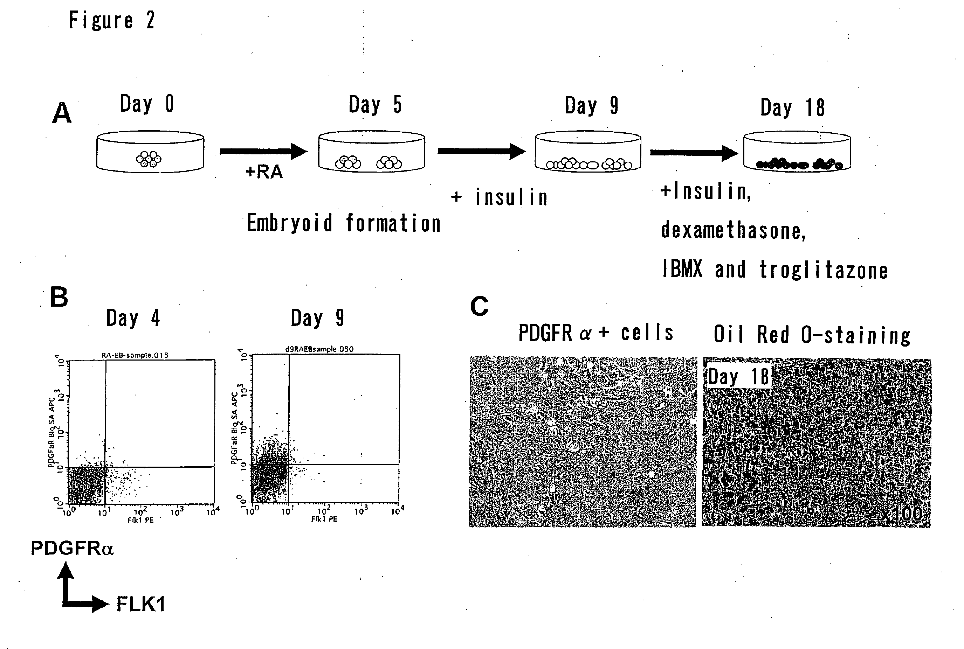

A mesenchymal stem cell of the present invention has a stroma cell-like morphology. A stroma cell is a star-shaped or spindle-shaped flat cell with a relatively large nucleus and a clearly defined nucleolus. Stroma cells closely resemble fibroblasts in form, but the cytoplasm is larger and somewhat rounder than that of a fibroblast, appearing typically as in Figure 2C (left) (Kodama, H.A. et al, *J. Cell Physiol.* 1982, 112, 89-95).

PDGFRα is a transmembrane receptor with tyrosine kinase activity in the intercellular part (Soriano, P., *Development* 124, 2691-2700, 1997). Its ligand is platelet-derived growth factor. Mice lacking PDGFRα have metameric and angiogenetic differentiation abnormalities. Like PDGFRα, FLK1 is a transmembrane receptor with tyrosine kinase activity in the intercellular part (Shalaby, F., *Cell* 89, 981-990, 1997). Its ligand is vascular endothelial growth factor (VEGF). Mice lacking FLK1 have abnormal differentiation of the blood and vascular endothelium, and die in embryo.

In these specifications, to be "positive" for a molecule means that a cell expresses that molecule, while to be "negative" means that the cell does not express it. FACS or the like can be used as described below to determined whether or not a cell expresses a molecule.

The mesenchymal stem cell of the present invention does not express genes such as PPARγ1, PPARγ2, adiponectin, UCP1 and the like which are known as fat cell markers, and does not express mesodermal markers such as Mesp2, DLL1, Lim1, Sox4, mesogenin and the like or nerve cell markers such as Otx1, Otx2 and the like (see Example 6). This means that the mesenchymal stem cell of the present invention is more differentiated than a mesodermal stem cell, but is at an earlier stage of differentiation than the 3T3L1 cell, a known fat precursor cell.

The inventors have already disclosed that when an embryonic stem cell is cultured on collagen IV to induce differentiation, PDGFRα-positive, FLK1-negative cells appear on day 3 after induction, reaching their maximum on day 4, and that these cells include mesodermal stem cells (Japanese Patent Application No. 2002-332232). The mesenchymal stem cell of the present invention is different from the aforementioned cells in that it can differentiate into fat cells, osteoblasts and/or cartilage cells (for example, see Example 4). The aforementioned cells express mesodermal markers such as Mesp2, DLL1, Lim1, Sox4, mesogenin and the like which are not expressed by the mesenchymal stem cell of the present invention. The mesenchymal stem cell of the present invention also differs from the aforementioned cells in terms of mode of expression of various other marker genes (see Example 5). In addition to these differences, the cell of the present invention is characterized by a stroma cell-like morphology.

The cell of the present invention and the cells of Japanese Patent Application No. 2002-332232 can be distinguished by looking at the presence or absence of mesodermal marker gene expression. It is enough to look at only the presence or absence of expression of the mesodermal marker gene Mesp2, but preferably the presence or absence of Lim1 and/or mesogenin is also investigated. Mesp2, Lim1 and mesogenin are all described in Saga, Y. et al, *Gene Dev.* 11, 1827-39, 1997; Tsang, T.E. et al, *Developmental Biology* 223, 77-90, 2000; and Yoon, J. et al, *Genes Dev.* 14, 3204-3214, 2000.

The mesenchymal stem cell of the present invention is used for transplantation into mammals or to obtain cells for transplantation into mammals. Transplantation can be to any mammals including humans.

In the method of preparing the mesenchymal stem cell of the present invention, ordinary operations and conditions in the technical field can be used for the specific operation of culturing the pluripotent stem cells. These can be determined appropriately for example after consulting Norio Nakatsuji Ed.: Experimental Medicine Supplement, Experimental Course for the Post-Genome Age 4, "Stem Cell and Clone Study Protocols," Yodosha (2001); Hogan, G. et al Eds.: Manipulating the Mouse Embryo: A Laboratory Manual, Cold Spring Harbor Laboratory Press, Plainview, NY (1994); and Robertson, E. J. Ed: Teratocarcinomas and Embryonic Stem Cells: A Practical Approach, IRL Press Oxford, UK (1987) and the like.

Typical subculturing operations and culture conditions are as follows using embryonic stem cells as an example. A dish is coated with gelatin, and embryonic stem cells are seeded thereon at a density of 10,000/cm² and cultured in an incubator at 37°C, 5% CO₂. The next day, the medium is replaced once, and once the cells have reached confluence on the second day they are rinsed once or twice with phosphate buffered saline and left for about five minutes after addition of a sufficient amount of 0.25% (w/v) trypsin-EDTA so as to cover the cell layer. The trypsin liquid is removed, a suitable amount of embryonic stem cell culture medium is added, and the cells are separated from the dish by pipetting. Cells are precipitated by normal centrifugal separation from this cell suspension. After removal of the supernatant, the precipitated cells are re-suspended in embryonic stem cell culture medium, seeded again to a concentration of 10,000/cm² on a gelatin-coated dish, and cultured.

Pluripotent stem cell maintenance medium is used to maintain the pluripotent cells in an undifferentiated state. For example, pluripotent stem cell maintenance medium is normally a minimal medium for cell culture with serum, LIF, L-glutamine, 2-mercaptoethanol and the like added thereto, and a sample composition is 85% Knockout D-MEM with 15% FBS, 10⁻⁴M 2-ME, 2 mM L-glutamine, 0.1 mM NEAA and 1000 U/ml LIF.

To differentiate the pluripotent stem cells, the pluripotent stem cells are cultured in pluripotent stem cell differentiation medium consisting of the aforementioned pluripotent stem cell maintenance medium with the LIF removed. A sample composition is 90% αMEM with 10% FBS, 5 x 10⁻⁵M 2-ME and 2 mM L-glutamine. When pluripotent stem cells are cultured in pluripotent stem cell differentiation medium, they begin to differentiate from an undifferentiated state into a variety of cells. When a number of culture days is mentioned in these specifications it signifies the number of days of culture in pluripotent stem cell differentiation medium.

Antibiotics and other substances useful for culture can be added to pluripotent stem cell maintenance medium and differentiation medium, and various substitutes that have the same function can be used in place of the various components of the medium. The various components of the medium are sterilized by methods suitable for each before use.

The method for preparing the mesenchymal stem cell of the present invention comprises confirming the appearance of stroma cells. In the present invention, the "appearance of stroma cells" means that cells with a stroma cell-like morphology constitute 1% or more or preferably 5% or more or more preferably 10% or more of the total cells. Under normal culture conditions in which an undifferentiated state is not maintained, cells with a stroma cell-like morphology begin to appear on about day 5 of culture. The morphology of the cells is generally observed through an optical microscope, phase contrast microscope, phase contrast inverted microscope or the like.

The method for preparing the mesenchymal stem cell of the present.invention also comprises taking some of the selected and separated cells and confirming the absence of expression of at least one mesodermal marker gene selected from Mesp2, Lim1 and mesogenin. The presence or absence of expression of the mesodermal marker gene can be confirmed by any method ordinarily used in the field, such as RT-PCR, Northern blotting, a method using a DNA chip, enzyme-linked immunosorbent assay (ELIZA) or antibody staining. Ordinarily, confirmation is preferably by RT-PCR.

Antibodies used for selecting and segregating cells in the method of preparing the mesenchymal stem cell of the present invention are polyclonal antibodies or monoclonal antibodies, and when using FACS as described below they are preferably monoclonal antibodies. Such antibodies can be prepared by someone skilled in the art with reference to the methods described in the examples, or commercial ones can be used. Anti-PDGFRα monoclonal antibodies (Product No. 558774) and anti-FLK1 monoclonal antibodies (Product No. 555308) are sold by BD Pharmingen and can be easily obtained.

In the method for preparing the mesenchymal stem cell of the present invention, a fluorescence activated cell sorter (FACS) is used for selecting and segregating cells using the respective cell surface markers. A FACS is normally equipped with a flow cytometer, a laser generator, optical system, a data processor and a cell sorter. The FACS function analyzes the autoseparation and fluorescent strength of fluorescent-labeled cells by means of a computer. With the FACS, cells which have been fluorescently labeled with a specific substance are exposed to laser light as they pass through a narrow passage, scattered light (forward scattered light or sideways scattered light) and fluorescence are measured as signal data for individual cells, the results are displayed for example as frequency distributions, and cells emitting specific signal data can be sorted. FACS units are sold by Becton-Dickinson and the like, and can be operated by someone skilled in the art according to the manufacturer's instructions.

A method using antibodies has been described in detail as a method of selecting cells in the present invention, but the presence of mRNA of each cell surface marker can also be used as the indicator for selection.

Step a) of the method for preparing the mesenchymal stem cell of the present invention may include a treatment to promote differentiation of the pluripotent stem cells. When a treatment is applied to cause differentiation into various mesenchymal cells, the target mesenchymal stem cells appear in the course of differentiation. Differentiation can be caused by any method known in the field. For example, in the case of embryonic stem cells, embryonic stem cells can be cultured on a collagen IV coated dish using embryonic stem cell differentiation medium containing 90% αMEM, 10% FBS, 5 x 10⁻⁵ M 2-ME and 2 mM L-glutamine, and incubated with trans-retinoic acid added to the culture medium on day 2 through day 4 of culture. Although efficiency is poor, the target cells will also appear if pluripotent cells are simply cultured on a collagen IV coated dish or gelatin coated dish.

In the method of the present invention, treatment to cause differentiation of pluripotent stem cells into fat cells can be adopted as the treatment to promote differentiation of the pluripotent stem cells. In the present invention, there is no need to completely differentiate the pluripotent stem cells into fat cells, since the target mesenchymal stem cells appear in the course of differentiation treatment. Differentiation can be caused by any method known in the field, but typically differentiation is caused by the methods of Jun Nakae et al as described in *Developmental Cell* 4, 119-129, 2003. For example, in the case of embryonic stem cells, pluripotent stem cells are cultured on a collagen IV coated dish using embryonic stem cell differentiation medium containing 90% αMEM, 10% FBS, 5 x 10⁻⁵ M 2-ME and 2 mM L-glutamine, and incubated with trans-retinoic acid added to the culture medium on day 2 through day 4 of culture and in some cases with insulin added beginning on day 5 or with insulin, dexamethasone, IBMX and troglitazone beginning on day 7 or day 11. They may also be differentiated after formation of the embryoid body as described by C. Dani in *Journal of Cell Science* 110, 1279-1285.

Alternatively, in the method of the present invention a treatment to differentiate the pluripotent stem cells into osteoblasts can be adopted as the treatment to promote differentiation of the pluripotent stem cells into mesenchymal stem cells. In the present invention there is no need to completely differentiate the pluripotent stem cells into osteoblasts, since the target mesenchymal stem cells appear in the course of the differentiation treatment. Differentiation can be by any method known in the field, and for example in the case of embryonic stem cells pluripotent stem cells are cultured on a gelatin coated dish using embryonic stem cell differentiation medium containing 90% αMEM, 10% FBS, 5 x 10⁻⁵ M 2-ME and 2 mM L-glutamine, and incubated with BMP-4, ascorbate-2-phosphate, dexamethasone and beta-glycerophosphate added to the culture medium beginning on day 2 of culture. Alternatively, the embryoid body can be formed and cultured for a long time awaiting the appearance of osteoblasts *(Methods Enzymol.* 365, 251-268, 2003).

In the method of the present invention, treatment to differentiate the pluripotent stem cells into cartilage cells can also be adopted as the treatment to promote differentiation of the pluripotent stem cells. In the present invention, there is no need to completely differentiate the pluripotent stem cells into cartilage cells, since the target mesenchymal stem cells appear in the course of differentiation treatment. Differentiation can be by any method known in the field, and for example in the case of embryonic stem cells the cells are incubated using embryonic stem cell differentiation medium comprising 90% αMEM, 10% FBS and 2 mM L-glutamine, with dexamethasone added to the culture medium beginning on day 2 of culture.

In the method for preparing the mesenchymal stem cell of the present invention, the occurrence of the PDGFRα-positive, FLK1-negative cells comprising mesodermal stem cells which have already been disclosed by the inventors (Japanese Patent Application No. 2002-332232) can be suppressed by the addition of trans-retinoic acid to the culture medium (see Example 1).

The present invention also provides a method for preparing fat cells that includes a treatment to differentiate the mesenchymal stem cell of the present invention into fat cells. As described in the examples, this treatment comprises for example the culturing of mesenchymal stem cells on a collagen IV coated culture dish in medium consisting of embryonic stem cell differentiation medium comprising 90% αMEM, 10% FBS and 2 mM L-glutamine to which has been added 5 µg/ml insulin, 1 µM dexamethasone, 500 µM 3-isobutyl-1-methylxanthine and 1 µM troglitazone. The fat cells are confirmed by cell staining using Oil Red.

The present invention also provides a method for preparing osteoblasts that includes a treatment to differentiate the mesenchymal stem cell of the present invention into osteoblasts. As described in the examples, this treatment comprises for example the culturing of mesenchymal stem cells on a gelatin coated dish in medium consisting of embryonic stem cell differentiation medium comprising 90% αMEM, 10% FBS and 2 mM L-glutamine to which has been added 10 ng/ml BMP-4, 50 µM ascorbate-2-phosphate, 0.1 µM dexamethasone and 10 mM beta-glycerophosphate. The osteoblasts are confirmed by cell staining using alizarin red.

The present invention also provides a method for preparing cartilage cells that includes a treatment to differentiate the mesenchymal stem cell of the present invention into cartilage cells. In the case of embryonic stem cells for example this treatment comprises the culturing of mesenchymal stem cells on medium comprising 90% αMEM, 10% FBS and 2 mM L-glutamine to which has been added 0.1 µM dexamethasone. The cartilage cells are confirmed by cell staining using alcian blue.

The present invention also provides a method for treating disorders of mammals that comprises the transplantation of mesenchymal stem cells, fat cells, osteoblasts and/or cartilage cells of the present invention into mammals. For example, large numbers of cartilage cells can be differentiated from the mesenchymal stem cell of the present invention and transplanted into joints as a means of treating diseases such as osteoarthritis of the knee joint in which the cartilage is destroyed. The mesenchymal stem cell of the present invention can also be injected intravenously to encourage myocardial regeneration in the treatment of myocardial infarction for example. The treatment methods of the present invention can be applied to any mammals including humans.

The present invention also provides a method of screening drugs using mesenchymal stem cells, fat cells, osteoblasts and/or cartilage cells of the present invention. For example, in the development of drugs for treating osteoporosis mesenchymal stem cells of the present invention can be cultured in medium with candidate substances added thereto to screen for substances which cause these cells to differentiate into osteoblasts.

The present invention is explained in detail below using examples, but these represent only one embodiment of the present invention and the present invention is not in any way limited thereby.

### Examples

### Preparation of mesenchymal stem cells differentiated from embryonic stem cells

### Reference Example 1. Maintenance of embryonic stem cells

### a. Materials

The reagents and tools shown in Table 1 were used for maintaining embryonic stem cells.

**Table 1**

| Reagents and tools | Maker | Product No. |
|---|---|---|
| Knockout Dulbecco's minimum essential medium (D-MEM) | Invitrogen | 10829-018 |
| Gelatin | Sigma | G2500 |
| 2-mercaptoethanol (2-ME) | Sigma | M7522 |
| Dulbecco's phosphate buffered saline MgCl₂(-), CaCl₂(-) | Invitrogen | 14190-250 |
| L-glutamine 200 mM | Invitrogen | 25030-081 |
| Non-essential amino acids (NEAA) | Invitrogen | 11140-050 |
| Penicillin-streptomycin (P/S) | Invitrogen | 15140-122 |
| Fetal bovine serum (FBS) | JRL | 8G4011 |
| LIF | Chemicon | ESG1107 |
| 0.25% (w/v) trypsin-EDTA | Invitrogen | 25200-072 |
| 6 cm dish | Falcon | 35 3802 |

The embryonic stem cell maintenance medium was composed of 85% Knockout D-MEM, 15% FBS, 10⁻⁴M 2-ME, 2 mM L-glutamine, 0.1 mM NEAA and 1000 U/ml LIF. CCE embryonic stem cells derived from 129sv mice were used as the embryonic stem cells (Robertson, E. et al, *Nature* 323, 445-448, 1986).

### b. Methods

The 6 cm dish was gelatin coated. 2 x 10⁵ of the CCE embryonic stem cells were seeded on this dish. The next day, the medium was replaced once. Once confluence was reached on the second day, the cells were separated from the dish with trypsin, and seeded again on a gelatin-coated dish at a density of 2 x 10⁵. Culture was in an incubator at 37°C, 5% CO₂.

### Reference Example 2. Differentiation of embryonic stem cells

### a. Materials

The reagents and tools shown in Table 2 were used to differentiate the embryonic stem cells.

| Reagents and tools | Maker | Product No. |
|---|---|---|
| Minimum essential alpha medium | Invitrogen | 12571-063 |
| 2-mercaptoethanol (2-ME) | Sigma | M7522 |
| Dulbecco's phosphate buffered saline MgCl₂(-), CaCl₂(-) | Invitrogen | 14190-250 |
| L-glutamine 200 mM | Invitrogen | 25030-081 |
| Penicillin-streptomycin (P/S) | Invitrogen | 15140-122 |
| Fetal bovine serum (FBS) | Invitrogen | US128311 |
| Biocoat collagen IV coated 10 cm dish | Becton-Dickinson | 35 4453 |
| Cell separation buffer | Invitrogen | 13150-016 |
| Trans-retinoic acid (RA) | Sigma | R2625 |
| For inducing fat cell differentiation | | |
| Insulin | Sigma | I5523 |
| Dexamethasone | Sigma | D2915 |
| 3-isobutyl-1-methylxanthine | Sigma | I7018 |
| Troglitazone | Sankyo | |
| For inducing osteoblast differentiation | | |
| BMP-4 | R&D | 314-BP-010 |
| Ascorbate-2-phosphate | Sigma | A-8960 |
| Dexamethasone | Sigma | D-8893 |
| Beta-glycerophosphate | Sigma | G-9891 |

| For inducing cartilage cell differentiation | | |
|---|---|---|
| Dexamethasone | Sigma | D-8893 |

The embryonic stem cell differentiation medium was composed of 90% αMEM, 10% FBS, 5 x 10⁻⁵ M 2-ME and 2 mM L-glutamine.

### b. Methods

3 x 10³ CCE embryonic stem cells were seeded on the Biocoat collagen IV coated 10 cm dish. The medium was replaced on day 5 and 7 using embryonic stem cell differentiation medium.

Fat cells were induced by addition of the reagents of Table 3 to embryonic stem cell differentiation medium containing no 2-ME. Culture was on a culture plate coated with collagen IV.

**Table 3**

| Reagent | Final concentration |
|---|---|
| Insulin | 5 µg/ml |
| Dexamethasone | 1 µM |
| 3-isobutyl-1-methylxanthine (IBMX) | 500 µM |
| Troglitazone | 1 µM |

Osteoblasts were induced by addition of the reagents of Table 4 to embryonic stem cell differentiation medium containing no 2-ME. Culture was on a culture plate coated with gelatin.

**Table 4**

| Reagent | Final concentration |
|---|---|
| BMP-4 | 10 ng/ml |
| Ascorbate-2-phosphate | 50 µM |
| Dexamethasone | 0.1 µM |
| Beta-glycerophosphate | 10 mM |

Cartilage cells were induced by addition of 0.1 µM dexamethasone to embryonic stem cell differentiation medium containing no 2-ME.

### Reference Example 3. Preparation of antibodies

Monoclonal antibodies that recognized the extracellular part of each molecule were prepared by methods well known to those skilled in the art-specifically, as follows. The cDNA of the extracellular part of mouse PDGFRα was amplified by PCR, and this DNA sequence was bound to the DNA sequence of the Fc part of human IgG1 to prepare fused cDNA. This cDNA was introduced into COS1 cells, and fused proteins in the culture supernatant were collected using a Protein A column. Rats were immunized with the collected protein. After completion of immunization, the spleens were collected and spleen cells were fused with myeloma cell strain X63.Ag8 to prepare hybridoma cells. To obtain the target antibodies, antibodies that reacted with Balb/c-3T3 cells expressing the fused protein and PDGFRα were selected from the antibodies contained in the hybridoma cell culture supernatant, and clones of the hybridoma cell were identified. Monoclonal antibodies that specifically recognized PDGFRα were obtainable by this method. Anti-mouse FLK1 monoclonal antibodies were prepared by roughly the same methods.

### Reference Example 4. Selection of cells by antibody staining and FACS Vantage

### a. Preparation of reagents

The reagents shown in Table 5 were used.

**Table 5**

| Reagent | Maker | Product No. |
|---|---|---|
| 10 x Hanks' balanced salt solution (10 x Hanks' buffer) | Invitrogen | 14185-052 |
| Bovine serum albumin (BSA) | Sigma | A-2153 |

100 ml of 10 x Hanks' buffer and 10 g of BSA (final concentration 1%) were added and to 900 ml of deionized water, and well agitated. Once the BSA had dissolved, this was filter sterilized with a 0.2 µm filter.

### b. Methods

Anti-PDGFRα antibodies were labeled with biotin and anti-FLK1 antibodies with phycoerythrin (both purchased from Molecular Probe), and used in the following staining. Cells on day 9 of differentiation were separated with cell separation buffer, and 10 µl/10⁶ cells of mouse serum was added and incubated for 20 minutes on ice. Next, 100 to 500 ng of each antibody was added, and incubated for 20 minutes on ice. Then, the cells were washed once with 1% BSA Hanks' buffer. The cells were re-suspended in 500 µl of 1% BSA Hanks' buffer containing streptavidin-allopycocyanine (APC; Becton-Dickinson), and incubated for 20 minutes on ice. Finally they were washed twice with 1% BSA Hanks' buffer, and 10⁶ cells were dissolved in 1 ml of 1% BSA Hanks' buffer and used in cell selection.

The FACS Vantage (Becton-Dickinson) and FacsAria (Becton-Dickinson) were used according to the attached guidelines. In the case of the FACS Vantage, the oscillating frequency of the nozzle was about 26,000, with a level of 3V and a drop delay of about 12 to 14.

### Example 1. Occurrence timing of PDGFRα-positive, FLK1-negative cells

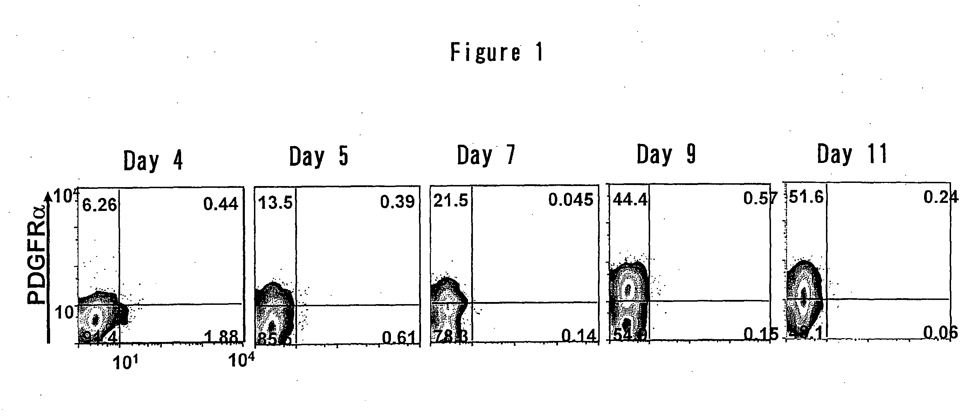

CCE embryonic stem cells were cultured in embryonic stem cell differentiation medium comprising 10⁻⁷ M of RA, and the progress of PDGFRα occurrence was investigated by FACS. Almost no PDGFRα-positive cells were found on day 4 of culture, but they appeared on day 5 and reached maximum by day 9 (Figure 1).

### Example 2. Separation of PDGFRα-positive, FLK1-negative cells

CCE embryonic stem cells were cultured and given a fat cell differentiation treatment as shown in Figure 2A. That is, CCE embryonic stem cells were cultured in medium consisting of embryonic stem cell differentiation medium with RA added from day 2 to day 4 of culture and insulin, dexamethasone, IBMX and troglitazone added beginning on day 11 to induce differentiation into fat cells. Expression of PDGFRα and FLK1 was studied over time by FACS. PDGFRα-positive, FLK1-negative cells appeared beginning on about day 8 or 9 after differentiation induction (Figure 2B). On day 9 of culture PDGFRα-positive, FLK1-negative cells were separated by FACS, and their morphology was observed by phase contrast microscopy. As shown in Figure 2C (left), the morphology of these cells was extremely similar to that of stroma cells, which are mesenchymal cells derived from bone marrow. These cells were cultured still further and the occurrence of fat cells was confirmed by fat staining (right: image of fat staining with Oil Red).

### Example 3. Ability of PDGFRα-positive, FLK1-negative cells to differentiate into fat cells

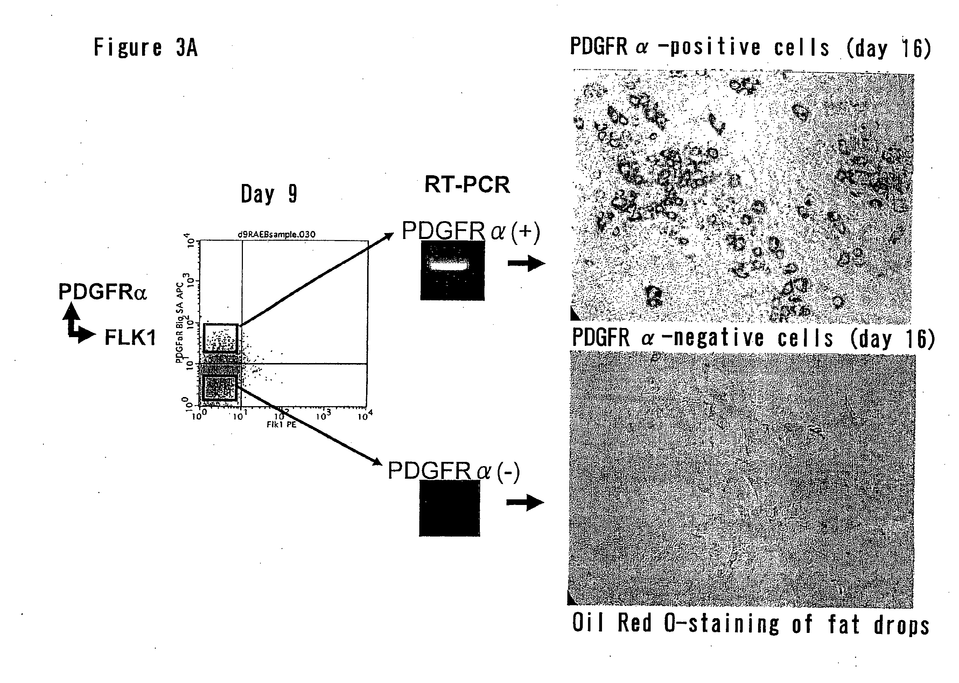

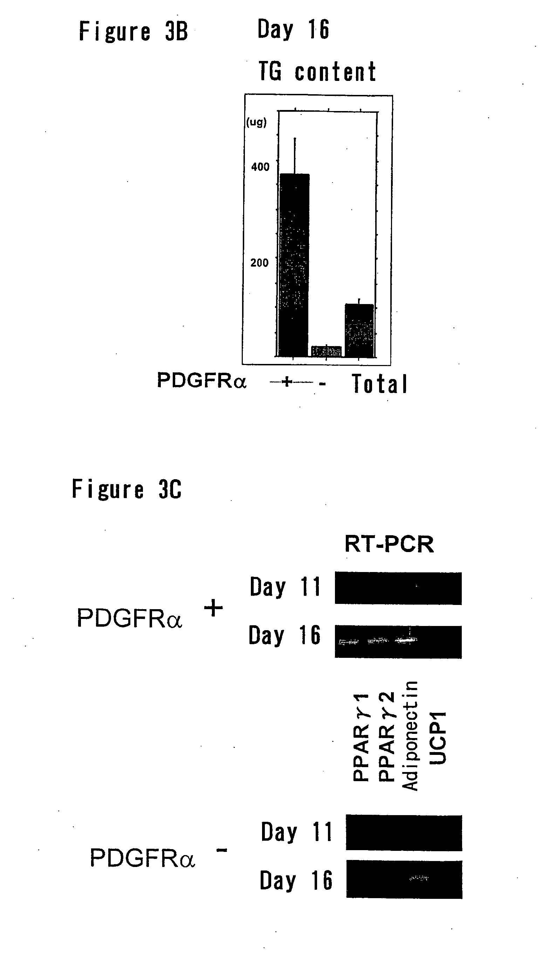

As in Example 2, CCE embryonic stem cells were cultured and given a fat cell differentiation treatment as shown in Figure 2A. On day 9 of culture, PDGFRα-positive, FLK1-negative cells were selected and separated using FacsVantage. After selection and separation these cells were cultured for 7 days and fat stained with Oil Red. As a result, it was shown that cells of the PDGFRα-positive, FLK1-negative fraction differentiated into fat cells with high efficiency (Figure 3A). These cells had large quantities of triglycerides, which are fat cell-specific substances (Figure 3B). The expression of fat-specific markers by cells that had been cultured for 3 days and 7 days after selection and separation was also investigated by RT-PCR, and it was confirmed that cells which had been cultured for 7 days after selection and separation expressed the fat-specific markers adiponectin and PPRγ (Figure 3C). When these cells were cultured after addition of dexamethasone and insulin to the medium, they differentiated efficiently into fat cells. PDGFRα-negative, FLK1-negative cells were selected and separated in the same way, cultured for 7 days, fat stained with Oil Red, and subjected to triglyceride measurement and RT-PCR. As shown in Figures 3A-C, fat cells did not occur from PDGFRα-negative cells.

### Example 4. PDGFRα-positive cells on day 4 of culture lack ability to differentiate into fat cells

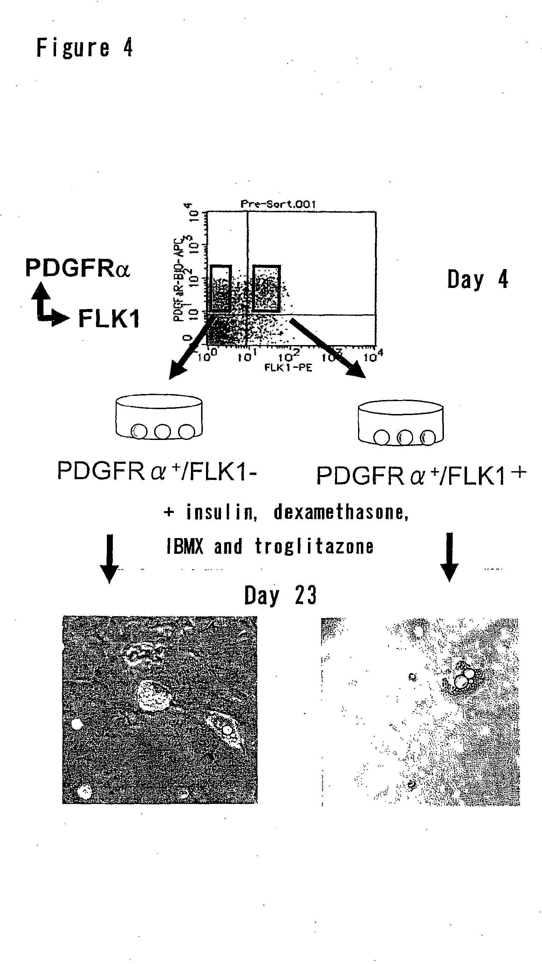

CCE embryonic stem cells were cultured in embryonic stem cell differentiation medium containing no RA, and PDGFRα-positive, FLK1-positive cells and PDGFRα-positive, FLK1-negative cells were selected and separated on day 4 of culture using FacsVantage. These cells were cultured for an additional 14 days (up to culture day 23) under the condition which induces differentiation into fat cells, and fat stained. As a result, the ability of both kinds of cells to differentiate into fat cells was shown to be extremely low (Figure 4).

### Example 5. Differences in gene expression between PDGFRα-positive, FLK1-negative cells on day 4 of culture and PDGFRα-positive, FLK1-negative cells on day 9 of culture

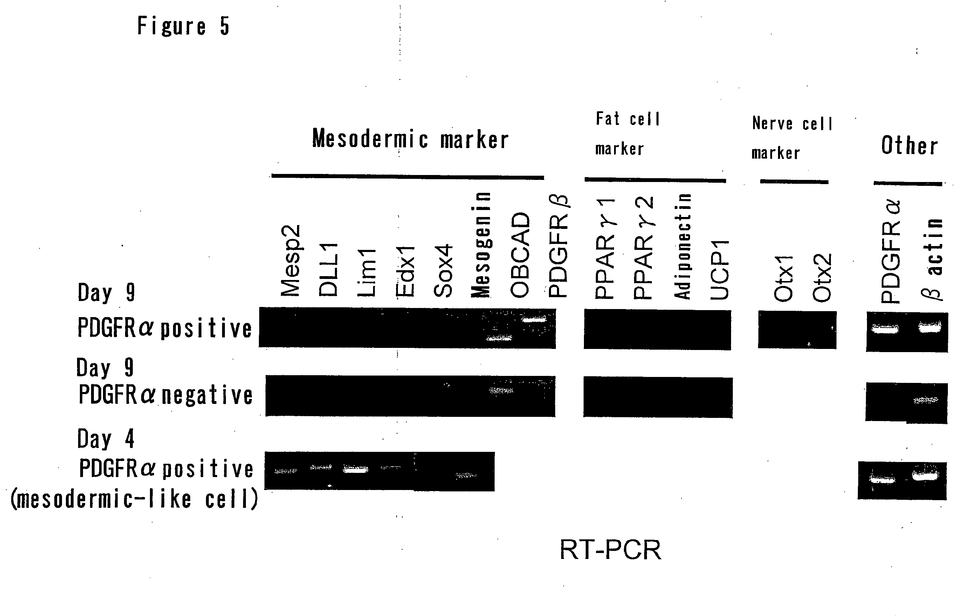

CCE embryonic stem cells were cultured in embryonic stem cell differentiation medium containing no RA, and PDGFRα-positive, FLK1-negative cells were selected and separated using FacsVantage on day 4 of culture. CCE embryonic stem cells were also cultured in embryonic stem cell differentiation medium containing 10⁻⁷ M RA, and PDGFRα-positive, FLK1-negative cells and PDGFRα-negative, FLK1-negative cells were selected and separated using FacsVantage on day 9 of culture. Expression of genes known as mesodermal cell markers was investigated in these cells by RT-PCR (Figure 5). While the RA(-) cells expressed mesodermal cell markers such as Lim1 on day 4, the RA(+) cells expressed no mesodermal cell markers on day 9 (Figure 5). This shows that PDGFRα-positive, FLK1-negative cells on day 9 of culture are different cells from PDGFRα-positive, FLK1-negative cells on day 4 of culture.

### Example 6. PDGFRα-positive, FLK1-negative cells do not express fat cell markers

CCE embryonic stem cells were cultured as in Example 2, and given a fat cell differentiation treatment as shown in Figure 2A. On day 9 of culture, the PDGFRα-positive, FLK1-negative cells were selected and separated using FacsVantage. Gene expression in these cells was investigated by RT-PCR. As shown in Figure 5, fat cell markers were not expressed, and neither were nerve cell markers. Judging from the cell morphology (see Example 2) and this gene expression pattern, it appears that these cells are at a stage of differentiation before fat cells.

### Example 7. RA addition on day 2 and day 3 of culture causes efficient differentiation of PDGFRα-positive, FLK1-negative cells

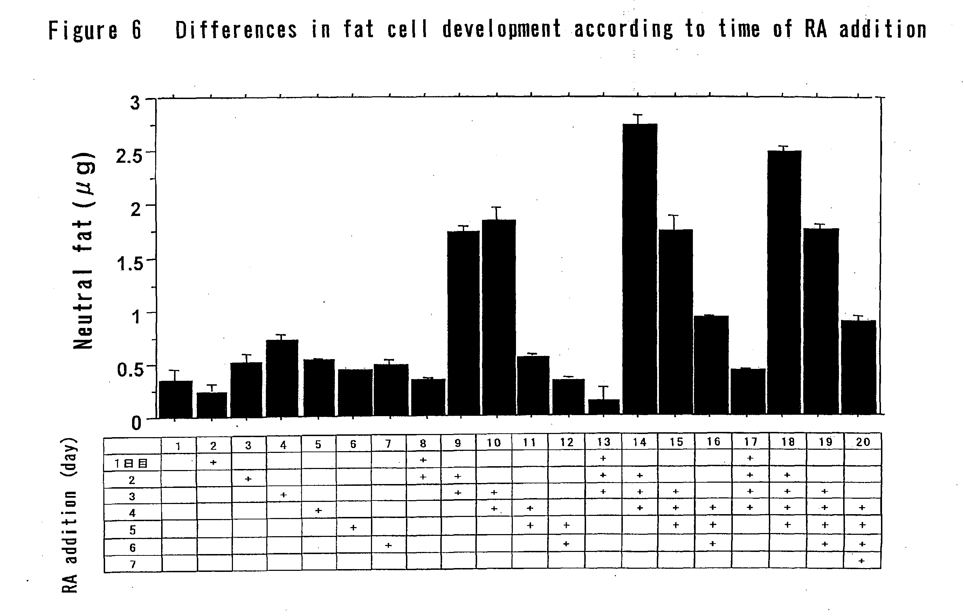

CCE embryonic stem cells were cultured in embryonic stem cell differentiation medium, RA was added at various times as shown in Figure 6, and the PDGFRα-positive, FLK1-negative cells were selected and separated on day 9 of culture using FacsVantage. These cells were cultured for 9 days under fat cell differentiation-inducing conditions, and the neutral fat content of the cells was measured. PDGFRα-positive, FLK1-negative cells appeared most efficiently when RA was added first on days 2, 3 and 4 after induction of embryonic stem cell differentiation (Figure 6). Differentiation into these cells was also induced when RA was added at other times, but efficiency was not as good as when addition was on days 2 through 4.

### Example 8. Autoreproducing ability of PDGFRα-positive, FLK1-negative cells

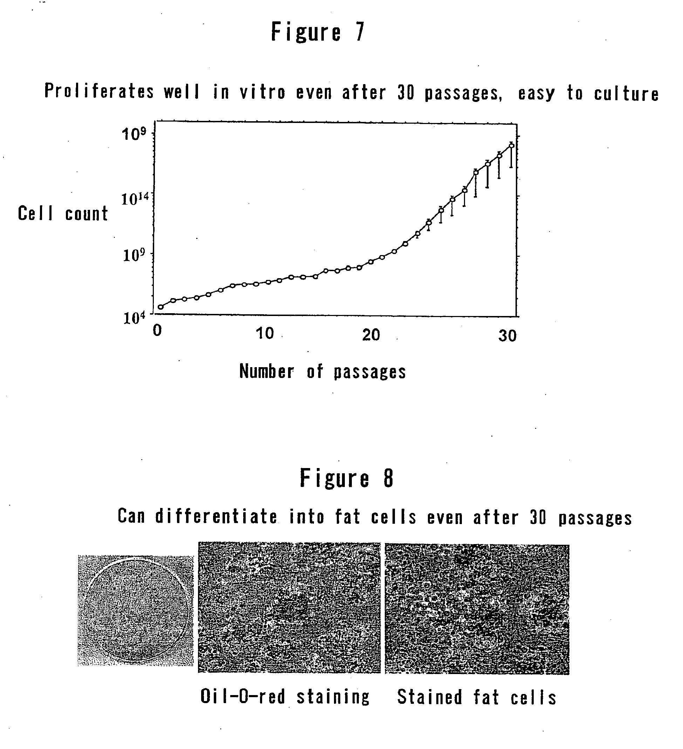

CCE embryonic stem cells were cultured as in Example 2, and given a fat cell differentiation treatment as shown in Figure 2A. On day 9 of culture the PDGFRα-positive, FLK1-negative cells were selected and separated using FacsVantage. These cells were subcultured 30 times in embryonic stem cell differentiation medium containing no 2-ME, and the cells were counted. As shown in Figure 7, even after 30 passages proliferation potency did not decline but actually increased. Cells from the 20^{th} and 30^{th} passage were cultured in fat cell differentiation medium. When these cells were fat stained with Oil Red, many were shown to have differentiated into fat cells as shown in Figure 8. This suggests that these cells have autoreproducing ability while maintaining the ability to differentiate.

### Example 9. Ability of PDGFRα-positive, FLK1-negative cells to differentiate into osteoblasts

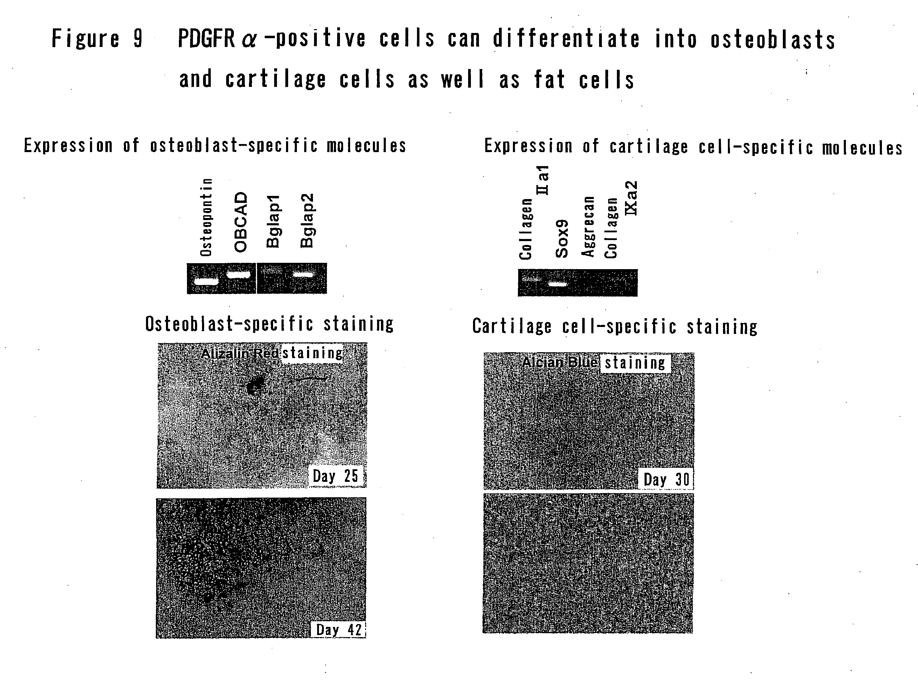

CCE embryonic stem cells were cultured as in Example 2 and given a fat cell differentiation treatment as shown in Figure 2A. On day 9 of culture the PDGFRα-positive, FLK1-negative cells were selected and separated using FacsVantage. These cells were cultured for 16 days (25 days total culture) under the osteoblast differentiation-inducing conditions described in Reference Example 2, and cells were collected and subjected to RT-PCR. These cells expressed osteoblast-specific molecules (Figure 9, upper left). These cells on the 25^{th} day of culture and cells that had been cultured through the 42^{nd} day were also stained with alizarin red, an osteoblast-specific staining agent (Figure 9, lower left). Most cells were stained, which supports that the PDGFRα-positive, FLK1-negative cells had differentiated into osteoblasts.

### Example 10. Ability of PDGFRα-positive, FLK1-negative cells to differentiate into cartilage cells.

CCE embryonic stem cells were cultured as in Example 2 and given a fat cell differentiation treatment as shown in Figure 2A. On day 9 of culture the PDGFRα-positive, FLK1-negative cells were selected and separated using FacsVantage. These cells were cultured for 21 days (30 days total culture) under the cartilage cell differentiation-inducing conditions described in Reference Example 2, and cells were collected and subjected to RT-PCR. These cells expressed cartilage cell-specific molecules (Figure 9, upper right). These cells on the 30^{th} day of culture were also stained with alcian blue, a cartilage cell-specific staining agent (Figure 9, lower right). Most of the cells were stained, which supports that the PDGFRα-positive, FLK1-negative cells had differentiated into cartilage cells.

### Example 11. Pluripotency of PDGFRα-positive, FLK1-negative cells

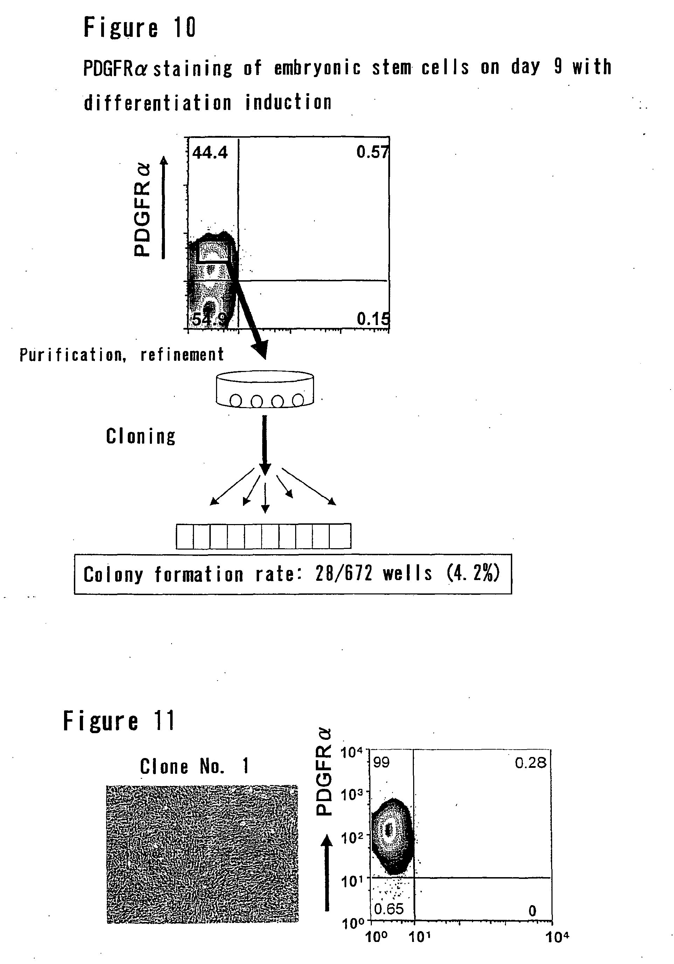

CCE embryonic stem cells were cultured as in Example 2 and given a fat cell differentiation treatment as shown in Figure 2A. On day 9 of culture the PDGFRα-positive, FLK1-negative cells were selected and separated using FacsVantage. These cells were subcultured 20 times. They were then seeded to a density of 0.3 cells/well on a flat-bottomed, 96-well plate, and cultured in embryonic stem cell differentiation medium. After the cells had proliferated and become confluent, the scale of the culture was increased from time to time. Finally they were proliferated on a 10 cm dish to an amount that could be cultured, and the cloned cell line was stored. 28 cloned cell lines were established (Figure 10). Each of these cell lines was PDGFRα-positive (Figure 11). Several attempts were made to directly clone cells after selection and separation, but these were unsuccessful because there was too much damage to the cells during the mechanical selection and separation operation.

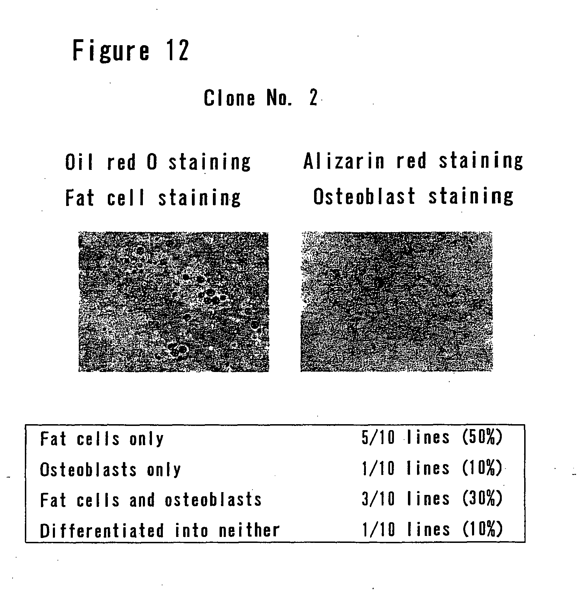

Of these cloned cell lines, 10 were cultured under conditions for differentiation into fat cells and osteoblasts. Each was fat cell stained and osteoblast stained (Figure 12). 50% of the cloned cell lines differentiated only into fat cells. 30% of the cloned cell lines differentiated into both fat cells and osteoblasts. 10% of the cloned cell lines differentiated only into osteoblasts.

As described above, because the PDGFRα-positive, FLK1-negative cells with a stroma cell-like morphology of the present invention have both pluripotency and the ability to autoreproduce, these cells are clearly mesenchymal stem cells.

### INDUSTRIAL APPLICABILITY

The mesenchymal stem cells of the present invention have the ability to differentiate into fat cells, osteoblasts, cartilage cells and other mesenchymal cells. The mesenchymal stem cells of the present invention and cells differentiated therefrom can be used in developing drugs that target these. For example, the fat cells are useful in developing drugs to treat diabetes and hyperlipidemia. Moreover, these cells can be used as cells which produce hormones, bioactive substances and the like, and can be applied to plastic surgical treatment and cell transplantation treatment such as mammaplasty using fat tissue. At present, the 3T3L1 cell line is known as a cell line which differentiates efficiently into fat cells, but the mesenchymal stem cells of the present invention have the advantage of being in an earlier stage of differentiation than 3T3L1 cells, which already express fat marker genes, and of not being a cancerated cell line. Moreover, the osteoblasts can be used to screen for drugs for treating and/or preventing osteoporosis. Basic research in regenerative medicine could also be performed by transplanting the osteoblasts and cartilage cells into experimental animals.

## Claims

1. A mesenchymal stem cell differentiated from a pluripotent stem cell in vitro, which is PDGFRα-positive and FLK1-negative and does not express Mesp2.

2. The mesenchymal stem cell according to claim 1, which also does not express Lim1 and/or mesogenin.

3. The mesenchymal stem cell according to claim 1 or 2, which has a stroma cell-like morphology.

4. The mesenchymal stem cell according to any of claims 1 through 3, wherein the pluripotent stem cell is derived from a mammal.

5. The mesenchymal stem cell according to any of claims 1 through 4, wherein the pluripotent stem cell is an embryonic stem cell.

6. The mesenchymal stem cell according to any of claims 1 through 5, for purposes of transplantation into mammals.

7. The mesenchymal stem cell according to any of claims 1 through 6, which is used for obtaining cells for purposes of transplantation into mammals.

8. A method for preparing a mesenchymal stem cell according to any of claims 1 through 7, comprising the following steps:
a) culturing a pluripotent stem cell,
b) confirming the occurrence of a cell with stroma cell-like morphology, and
c) selecting and segregating cells which are PDGFRα-positive and FLK1-negative.

9. The method according to claim 8, further comprising
d) taking some of the cells which are selected and separated in the step c), and confirming absence of expression of at least one mesodermal marker gene selected from the group consisting of Mesp2, Lim1 and mesogenin.

10. The method according to claim 8 or 9, wherein the step c) is performed on or after day 5 of culture.

11. The method according to any of claims 8 through 10, wherein anti-PDGFRα antibodies and/or anti-FLK1 antibodies are used in the step c).

12. The method according to any of claims 8 through 11, wherein cells are selected and separated by FACS in the step c).

13. The method according to any of claims 8 through 12, wherein the pluripotent stem cells are treated to promote differentiation in the step a).

14. The method according to claim 13, wherein said treatment comprises culturing the pluripotent stem cells on a culture plate coated with collagen IV.

15. The method according to claim 13 or 14, wherein said treatment comprises the addition of at least one substance selected from trans-retinoic acid, insulin, dexamethasone, IBMX and troglitazone to the culture medium.

16. The method according to claim 15, wherein the substance is trans-retinoic acid.

17. A method for preparing fat cells, comprising treatment to differentiate mesenchymal stem cells according to any of claims 1 through 7 into fat cells.

18. The method according to claim 17, wherein said treatment comprises culturing the mesenchymal stem cells on a culture plate coated with collagen IV.

19. The method according to claim 17 or 18, comprising the addition of at least one substance selected from trans-retinoic acid, insulin, dexamethasone, IBMX and troglitazone to the culture medium.

20. A method for preparing osteoblasts, comprising treatment to differentiate mesenchymal stem cells according to any of claims 1 through 7 into osteoblasts.

21. The method according to claim 20, wherein said treatment comprises culturing the mesenchymal stem cells on a culture plate coated with gelatin.

22. The method according to claim 20 or 21, comprising the addition of at least one substance selected from BMP-4, ascorbate-2-phosphate, dexamethasone and β-glycerophosphate to the culture medium.

23. A method for preparing cartilage cells, comprising treatment to differentiate mesenchymal stem cells according to any of claims 1 through 7 into cartilage cells.

24. The method according to claim 23, comprising the addition of dexamethasone to the culture medium.

25. A mesenchymal stem cell obtainable by a method according to any of claims 8 through 16.

26. A fat cell obtainable by a method according to any of claims 17 through 19.

27. An osteoblast obtainable by a method according to any of claims 20 through 22.

28. A cartilage cell obtainable by a method according to claim 23 or 24.

29. A method for treating disorders of mammals, comprising the transplantation of a cell according to any of claims 1 through 7 and claims 25 through 28 to a mammal.

30. A method for screening drugs using a cell according to any of claims 1 through 7 and claims 25 through 28.
